Europäisches Patentamt

European Patent Office

Office européen des brevets

(19)

(11) Veröffentlichungsnummer: 0 368 264

A1

(12) EUROPÄISCHE PATENTANMELDUNG

(21) Anmeldenummer: 89120644.3

(22) Anmeldetag: 08.11.89

(51) Int. Cl.5: A61M 35/00, A61F 13/02

(30) Priorität: 10.11.88 DE 3838061

(43) Veröffentlichungstag der Anmeldung:
16.05.90 Patentblatt 90/20

(84) Benannte Vertragsstaaten:
CH DE FR GB IT LI

(71) Anmelder: Holzer, Walter, Senator h.c.
Dr.h.c.Ing.
Drosteweg 19
D-7758 Meersburg(DE)

(72) Erfinder: Holzer, Walter, Senator h.c.
Dr.h.c.Ing.
Drosteweg 19
D-7758 Meersburg(DE)

(74) Vertreter: Riebling, Peter, Dr.-Ing.,
Patentanwalt
Rennerle 10, Postfach 31 60
D-8990 Lindau/B.(DE)

(54) Sterile Selbstklebefolie für die medizinische Eingriffsvorbereitung.

(57) Eine sterile Selbstklebefolie (1) dient zur Abdeckung des Bereichs für einen transcutanen Eingriff in der Tier- oder Humanmedizin. Die Selbstklebefolie weist mindestens eine Trägerfolie auf, die mindestens eine klebende Fläche (3) aufweist, die direkt auf die zu behandelnde Haut (10) aufgeklebt wird. Die klebende Fläche ist ganz oder teilweise mit bioaktiven Substanzen imprägniert, z. B. mit schmerzbetäubenden Substanzen, keimreduzierenden Substanzen, mit Substanzen, die die Durchblutung des Bereichs des transcutanen Eingriffes fördern.

FIG 3

EP 0 368 264 A1

## Sterile Selbstklebefolie für die medizinische Eingriffsvorbereitung

Gegenstand der vorliegenden Erfindung ist eine sterile Selbstklebefolie für die medizinische Eingriffsvorbereitung, insbesondere für chirurgische Zwecke.

Es sind zwar sogenannte Erste-Hilfe-Heftpflaster bekanntgeworden (DE 34 39 239, DE 35 43 263); bei diesen Heftpflastern handelt es sich doch um Wundverbände. Der Einsatz eines derartigen Wundverbandes für die medizinische Eingriffsvorbereitung am menschlichen oder tierischen Körper ist jedoch nicht möglich.

Das Problem des Hospitalismus ist in der Human- und Tiermedizin wohl bekannt. Zwar wird bei der Vorbereitung einer Hautfläche zur Vornahme eines Eingriffes die betreffende Hautstelle gereinigt und desinfiziert. Derartige Eingriffe können sowohl chirurgischer Art sein als auch das einfache Verabreichen von Spritzen.

Das Reinigen und Desinfizieren der für den Eingriff vorbereiteten Hautstelle gelingt nicht immer ausreichend. Damit besteht dann die Gefahr, daß bei der Durchführung des chirurgischen Eingriffes oder beim Setzen der Spritze nicht abgetötete Keime mit dem Einstich oder dem Schnitt mit in die geöffnete Blutbahn gelangen und zu einer Sepsis führen können.

Bei leichteren chirurgischen Eingriffen, die lediglich unter lokaler Betäubung erfolgen, gelingt nicht immer eine ausreichende Schmerzlinderung an der Eingriffsstelle.

Bisher sind nämlich lediglich Selbstklebefolien in der Chirurgie bekannt, die durch einen Fensterausschnitt ein Operationsfeld definieren. Eine medizinisch aktive Einwirkung dieser bekannten Selbstklebefolien auf das Operationsfeld ist jedoch nicht bekannt.

Der vorliegenden Erfindung liegt deshalb die Aufgabe zugrunde eine Selbstklebefolie der eingangs genannten Art so weiterzubilden, daß die Haut an der Eingriffsstelle mit zusätzlichen medizinisch wirksamen Substanzen beeinflußt werden kann, um einen transcutanen Eingriff durch die Folie hindurch zu ermöglichen.

Zur Lösung der gestellten Aufgabe ist die Erfindung dadurch gekennzeichnet, daß an der sterilen Selbstklebefolie mindestens eine klebende Fläche angeordnet ist, die auf die zu behandelnde Hautschicht aufgeklebt wird und daß diese klebende Fläche ganz oder teilweise mit einer medizinisch wirksamen Substanz imprägniert ist.

Mit der gegebenen technischen Lehre wird sichergestellt, daß im Eingriffsgebiet auf der Haut keine schädlichen Bakterien mehr durch das Instrument (Messer oder Spritze oder dgl.) in die Haut eingeschleust werden können.

In einer bevorzugten Ausführungsform ist vorgesehen, daß die klebende Fläche ganz oder teilweise mit einer schmerzbetäubenden Substanz imprägniert ist. Damit kann eine derartige Selbstklebefolie günstig für kleinere Eingriffe verwendet werden, bei denen eine Schmerzreduzierung im Bereich der Eingriffsstelle an der Hautoberfläche erwünscht ist.

Von besonderem Vorteil wird eine derartige Selbstklebefolie bei der Verabreichung von Spritzen eingesetzt. Es entfällt damit die Reinigung mit einem getränkten Wattebausch, der den Nachteil hat, daß vorhandene Keime über die gesamte Fläche verschleppt werden und beim Eindringen der Kanüle in die Haut mit in die Haut eingetragen werden.

Nach der Erfindung wird eine Selbstklebefolie verwendet, deren klebende Fläche mit einer schmerzbetäubenden Substanz imprägniert ist und damit wird der Einstich der Kanüle in die Haut kaum mehr gespürt.

Darüber hinaus ist es in weiteren Fortbildungen der Erfindung vorgesehen, daß die klebende Fläche nicht nur mit der schmerzbetäubenden Substanz imprägniert ist, sondern zusätzlich eine keimreduzierende Substanz enthält oder noch andere Substanzen nach den Gegenständen der Unteransprüche 3 bis 7.

Nach dem Aufkleben der Selbstklebefolie auf das betreffende Hautgebiet wandern die schmerzbetäubenden Substanzen in die Hautschichten, wo sie die Nervenenden erreichen. Neben einer Schmerzbetäubung ist gleichzeitig eine Keimreduzierung im Eingriffsgebiet gegeben. Die Selbstklebefolie umschließt die Kanüle ringförmig und streift an der Außenseite der Kanüle noch eventuell sitzende Verunreinigungen ab, die damit in der Selbstklebefolie verbleiben.

Nachdem die Folie bevorzugt transparent ausgebildet ist kann man den Einstichbereich gut übersehen.

Durch das Aufkleben der Folie werden - soweit gefährliche Bakterien auf der Haut sitzen - diese festgeklebt und unschädlich gemacht.

Das Einstechen der Kanüle in die Folie wirkt so, als wenn man in eine elastische Membrane einsticht, so daß - wie oben stehend beschrieben - die Kanüle noch zusätzlich gesäubert wird.

Besonderer Bedeutung kommt auch der Eigenschaft der elastischen Folie zu, die Einstichöffnung der Kanüle unmittelbar nach dem Herausziehen wieder zu schließen, so daß ein nachträgliches Anbringen eines Verbandstreifens unterbleiben kann.

Nachdem also die Klebefolie im Bereich der Einstichsstelle eine sich verformende Ringzone bildet, verformt sich diese Ringzone nach dem Herausziehen der Kanüle wieder zurück, wodurch die Folie praktisch hermetisch geschlossen wird und ein Ausbluten der Wunde unterbunden wird.

Die erfindungsgemäße Selbstklebefolie dient dann auch als Wundverband.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander. Alle in den Unterlagen - einschließlich der Zusammenfassung -offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von mehreren Ausführungsbeispielen darstellenden Zeichnungen näher erläutert. Hierbei gehen aus den Zeichnungen und ihrer Beschreibung weitere erfindungswesentliche Merkmale und Vorteile der Erfindung hervor.

Es zeigen:

Figur 1 perspektivisch eine sterile Selbstklebefolie in einer ersten Ausführungsform;

Figur 2 eine Selbstklebefolie in einer zweiten Ausführungsform mit dazugehörender Verpackung;

Figur 3 die Anwendung einer Selbstklebefolie nach Fig. 1 oder 2 auf der Haut beim Setzen einer Spritze.

Die eigentliche transparente Selbstklebefolie 1 ist mit der imprägnierten Selbstklebeschicht 3 bedeckt, welche durch die Schutzfolie 5 gegen Verunreinigungen bis kurz vor der Benutzung geschützt wird. Auf der Oberseite der Selbstklebefolie 1 befindet sich eine sterile Haftschicht 2, auf der eine Andrückfolie 4 leicht lösbar klebt. Die Haftschicht 2 kann auch an der Unterseite der Andrückfolie 4 angeordnet sein. Dieses Paket von drei Folien stellt das Grundelement der Erfindung dar und gestattet einen einwandfreien Einsatz oder eine einwandfreie Benutzung wie folgt:

Zunächst wird kurz vor dem Aufkleben auf die Haut die Schutzfolie 5 von der Selbstklebefolie 1 abgezogen, was dadurch erleichtert wird, daß die imprägnierte Selbstklebeschicht 3 nicht über die ganze Fläche der Folie 1,5 reicht.

Die Selbstklebefolie 1 kann nun auf die Hautoberfläche angedrückt werden, wobei die Andrückfolie 4 bei diesem Vorgang die Selbstklebefolie sauber hält. Die Andrückfolie 4 bleibt nun so lange auf der Selbstklebefolie 1, bis kurz vor dem Einstich der Injektionsnadel dieselbe abgezogen wird und eine einwandfreie sterile Außenfläche der Haftschrift 2 garantiert. Das Abziehen der Andrückfolie 4 wird ebenfalls dadurch erleichtert, daß die Haftschicht 2 nicht über die ganze Oberfläche der Selbstklebefolie 1 reicht, so daß ein nicht-klebender Griffrand gebildet wird.

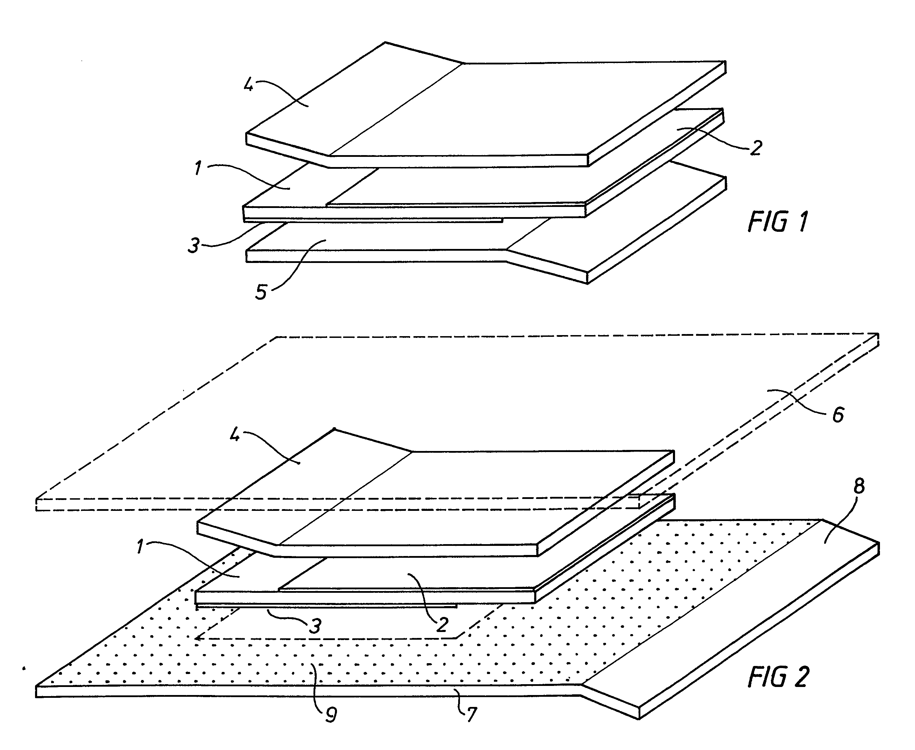

Eine derart geschützte Selbstklebefolie gemäß Figur 1 muß selbstverständlich eine zusätzliche Verpackung erhalten, die nicht dargestellt ist.

In Figur 2 ist die Folie 7 gleichzeitig als Außenverpackung gedacht. Sie ist in der Fläche größer als die Selbstklebefolie 1, die auch in diesem Fall nur mit der Fläche 8 auf der Folie 7 haftet. Die Andrückfolie 4 hat die gleiche Aufgabe wie in Figur 1, aber es ist noch zusätzlich eine

Verpackungsfolie 6 vorgesehen, welche nur am Außenrand 9 mit der Folie 7 haftet.

Bei dieser Ausführung wird zunächst die sterile Verpackung, bestehend aus den Folien 6,7 gelöst bzw. geöffnet, sodann die Folie 7 von der Selbstklebefolie 1 kurz vor Anbringung auf der Haut abgezogen, wobei die Andrückfolie 4, wie in Figur 1 beschrieben, erst unmittelbar vor dem Eingriff, d.h. durch Stechen mit der Injektionsnadel abgezogen wird.

Selbstverständlich ist es erfindungsgemäß auch denkbar, die Andrückfolie 4 größer auszuführen und als obere Verpackungsfolie zu gestalten, wobei die Verpackungsfolie 6 entfällt.

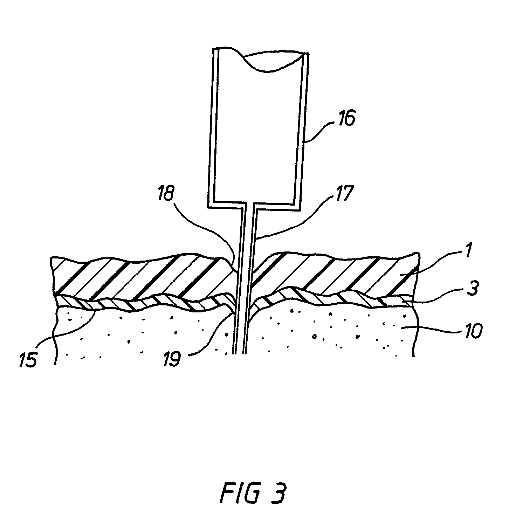

Die Figur 3 zeigt als vereinfachtes Ausführungsbeispiel das Eindringen einer Kanüle 17 einer Spritze 16 in eine Einstichsöffnung 18 der menschlichen Haut. Hierbei ist die Trägerfolie 1 mit ihrer unteren, klebenden Fläche 3 auf die Oberhaut 10 aufgeklebt. In bekannter Weise schließt sich an die Oberhaut 10 die Hornschicht 12 und darunter die Keimschicht 13 an. Daran schließt sich die Lederhaut 11 an. Der untere Bereich der Lederhaut 11 wird durch das Unterhaut-Zellgewebe 14 gebildet. Im vorliegenden Ausführungsbeispiel erreicht die Kanüle 17 lediglich bis in das Unterhaut-Zellgewebe 14.

Man erkennt, daß durch Einstich der Kanüle 17 in die Trägerfolie 1 sich eine kraterförmige Einstichsöffnung 18 ergibt, die eine gleichfalls kraterförmige, nach unten in Richtung zur Oberhaut 10 weisende Ringzone 19 aufweist.

Diese Ringzone dringt teilweise mit in die Oberhaut 10 hinein. Dadurch wird gewährleistet, daß die mit den bioaktiven Substanzen imprägnierte klebende Fläche 3 die Substanzen über die kraterförmig sich in die Haut 10 hinein erstreckende Ringzone 19 in die Haut eingeschleust werden. Die Einstichstelle 18,19 wird damit optimal gegen äußere Einflüsse abgedichtet.

Zusätzlich wird gewährleistet, daß die durch die klebende Fläche 3 sich auf der Haut 10 ergebende Kontaktfläche 15 gegen Eindringen von Bakterien von der Seite her abgedichtet wird und zusätzlich ist erfindungsgemäß vorgesehen, daß die klebende Fläche 3 mit entsprechenden keimreduzierenden Substanzen imprägniert ist, so daß die dort im Bereich der Kontaktfläche 15 befindlichen Bakterien festgehalten und unschädlich gemacht werden. Sie können dadurch nicht in die Blutbahn im Bereich der Einstichsstelle 18,19 gelangen.

Nach dem Entfernen der Kanüle 17 aus der Einstichsstelle 18,19 verformt sich die Ringzone 19 wieder nach außen, so daß sich hierdurch ein vollständig abgeschlossener Bereich der klebenden Fläche 3 ergibt, ebenso wie sich die Einstichsöffnung 18 der Trägerfolie 1 wieder zurückverformt und sich hierdurch wieder vollständig schließt. Damit wird die Einstichsstelle 18,19 in die Haut gegen Blutungen abgedichtet.

Ferner wird ein Eindringen von Bakterien von oben oder von der Seite zuverlässig verhindert. Jetzt dient die Trägerfolie also als Wundverband, der ein Eindringen von Bakterien zuverlässig verhindert.


## ZEICHNUNGS-LEGENDE


1 Selbstklebefolie
2 Haftschicht
3 imprägnierte Selbstklebeschicht
4 Andrückfolie
5 Schutzfolie
6 Verpackungsfolie
7 Folie
8 Fläche
9 Außenwand
10 Haut
15 Kontaktfläche
16 Spritze
17 Kanüle
18 Einstichöffnung
19 Ringzone


**Ansprüche**

1. Sterile Selbstklebefolie zur Abdeckung des Bereichs für einen transcutanen Eingriff, beispielsweise eine Injektion, **dadurch gekennzeichnet,** daß die auf die Haut aufgelegte Fläche (2,3) ganz oder teilweise mit einer medizinisch wirksamen Substanz imprägniert ist.

2. Sterile Selbstklebefolie nach Anspruch 1, **dadurch gekennzeichnet,** daß die medizinisch wirksame Substanz ein Anästetikum ist.

3. Sterile Selbstklebefolie nach Anspruch 1 oder 2, **dadurch gekennzeichnet,** daß die Fläche (2,3) mit einer keimreduzierenden Substanz imprägniert ist.

4. Sterile Selbstklebefolie nach Anspruch 1 bis 3, **dadurch gekennzeichnet,** daß die Fläche (2,3) mit einer die Durchblutung des Bereiches des transcutanen Eingriffes fördernden Substanz imprägniert ist.

5. Sterile Selbstklebefolie nach einem der Ansprüche 1 bis 4, **dadurch gekennzeichnet,** daß die Fläche (2,3) zusätzlich mit einer Substanz imprägniert ist, welche das Eindringen der anderen aktiven Substanzen in die Haut begünstigt.

6. Sterile Selbstklebefolie nach einem oder mehreren der vorstehenden Ansprüche, **dadurch gekennzeichnet,** daß die Selbstklebefolie (1) nur in einem umlaufenden Randbereich mit einer Klebeschicht ausgerüstet ist.

7. Sterile Selbstklebefolie nach einem der Ansprüche 1 - 5, **dadurch gekennzeichnet,** daß die Selbstklebefolie (1) auf der gesamten Fläche einschließlich der Fläche, welche mit den medizinischen Substanzen imprägniert ist, mit einer Klebeschicht versehen ist.

8. Sterile Selbstklebefolie nach einem oder mehreren der vorstehenden Ansprüche 1 - 7, **dadurch gekennzeichnet,** daß die Selbstklebefolie (1) und/oder die Substanzen der Fläche (2,3) antiallergisch sind.

9. Sterile Selbstklebefolie nach einem oder mehreren der vorstehenden Ansprüche 1 - 8, **dadurch gekennzeichnet,** daß alle/oder einige der Substanzen nach der Applikation resorbierbar und/oder abbaubar sind.

10. Sterile Selbstklebefolie nach einem oder mehreren der vorstehenden Ansprüche 1 - 9, **dadurch gekennzeichnet,** daß die Selbstklebefolie (1) elastisch ist.

11. Sterile Selbstklebefolie nach Anspruch 8, **dadurch gekennzeichnet,** daß die Selbstklebefolie (1) eine elastische Faserstruktur besitzt.

12. Sterile Selbstklebefolie nach einem oder mehreren der vorstehenden Ansprüche 1 - 11, **dadurch gekennzeichnet,** daß die Selbstklebefolie (1) mehrschichtig mit einer oder zwei Abdeckfolien (6,7) ausgestattet ist.

13. Sterile Selbstklebefolie nach Anspruch 12, **dadurch gekennzeichnet,** daß eine oder beide Abdeckfolien (6,7) als Teil der Außenverpackung ausgebildet sind.

FIG 1

FIG 2

EP 0 368 264 A1

FIG 3

H 24(

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | GB-A- 944 329 (Blackford) <br> * Seite 2, Zeilen 11-32 * <br> --- | 1-3,7, 10 | A 61 M 35/00 <br> A 61 F 13/02 |
| Y | FR-A-2 009 721 (Weinz) <br> * Seite 1, Zeilen 4-21 * <br> --- | 1-3,7, 10 | |
| A | EP-A-0 258 521 (Latzke) <br> * Spalte 5, Zeilen 15-27 * <br> --- | 4 | |
| A | EP-A-0 272 918 (Cygnus) <br> * Spalte 6, Zeilen 32-44 * <br> --- | 5 | |
| A | US-A-4 402 696 (Gulko) <br> * Spalte 2, Zeilen 4-16,28-34; Fig 1 * <br> --- | 6,12,13 | |
| A | US-A-4 781 966 (Taylor) <br> * Spalte 8, Zeilen 9-12 * <br> ----- | 11 | |

RECHERCHIERTE SACHGEBIETE (Int. Cl.5)

A 61 M
A 61 F

Der vorliegende Recherchenbericht wurde für alle Patentansprüche erstellt

| Recherchenort | Abschlußdatum der Recherche | Prüfer |
|---|---|---|
| DEN HAAG | 09-01-1990 | MOERS R.J. |